(19) Europäisches Patentamt
European Patent Office
Office européen des brevets

(11) Numéro de publication: **0 290 319**
**B1**

(12) # FASCICULE DE BREVET EUROPEEN

(45) Date de publication du fascicule du brevet: **08.08.90**

(51) Int. Cl.[5]: **C07D 319/14,** C07C 43/313

(21) Numéro de dépôt: **88401028.1**

(22) Date de dépôt: **27.04.88**

(54) **Fluoro-1 Halogéno-1 dioxa-3,6 bicyclo [4.1.0] heptane, procédé de préparation et utilisation.**

(30) Priorité: **06.05.87 FR 8706600**

(43) Date de publication de la demande:
**09.11.88 Bulletin 88/45**

(45) Mention de la délivrance du brevet:
**08.08.90 Bulletin 90/32**

(84) Etats contractants désignés:
**AT BE CH DE ES FR GB GR IT LI LU NL SE**

(56) Documents cités:
**DE-A- 2 016 990**

**LIEBIGS ANNALEN DER CHEMIE, vol. 7, 1982, pages 1348-1358, Verlag Chemie GmbH, Weinheim, DE; R. DERSCH et al.: "Synthesen mit aliphatischen Dialdehyden, XXXIV. Versuche zur Darstellung von Brom-(Chlor-)fluormalonaldehyd sowie von Fluor(methyl)-und Fluor(phenyl)malonaldehyd"0, 40, 102-6n-hexylocatanamides", & COM- NAZ. ENERG. NUCL., [RAPP. TEC.] CN 000**

(73) Titulaire: **RHONE-POULENC CHIMIE, 25, quai Paul Doumer, F-92408 Courbevoie Cédex(FR)**

(72) Inventeur: **Molines, Huguette, 10, rue des Jardiniers, F-75012 Paris(FR)**
Inventeur: **Wakselman, Claude, 5, rue Chanez, F-75016 Paris(FR)**

(74) Mandataire: **Le Pennec, Magali et al, RHONE-POULENC INTERSERVICES Service Brevets Chimie 25, Quai Paul Doumer, F-92408 Courbevoie Cédex(FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**EP 0 290 319 B1**

ACTORUM AG

## Description

La présente invention concerne une classe de produits nouveaux les fluoro-1 halogéno-1 dioxa-3,6 bicycloheptanes. Elle concerne plus particulièrement la classe des dérivés ci-dessus dans laquelle le motif halogène représente le chlore ou le fluor et encore plus préférentiellement le chloro-1 fluoro-1 dioxa-3,6 bicyclo [4.1.0.] heptane. Elle concerne aussi le procédé de préparation de ces composés et leurs utilisations.

Ces composés sont en effet utilisés pour la préparation de fluoromalonaldéhyde ou des fluoromalonates d'alkyle utilisés eux-mêmes pour la synthèse d'acides aminés et d'hétérocycles fluorés tels que notamment le fluorouracile. Le fluoromalonaldéhyde est lui-même utilisé par exemple selon le brevet DE 2 016 990 pour la préparation d'hétérocycles azotés substitués par un atome de fluor présentant des propriétés anticancéreuses, antinéoplastiques ou antibactériennes.

Il est connu selon le brevet DE 2017010 de préparer du fluoromalonaldéhyde de formule

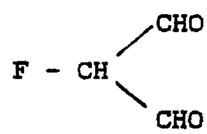

par hydrolyse alcaline ou alcalino-terreuse de la fluoro-2 diméthylamino-3 acroléine de formule

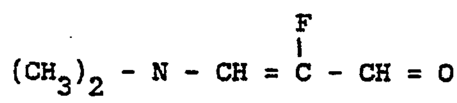

Pour préparer la fluoro-2 diméthylamino-3 acroléine il est nécessaire de mettre en présence du fluoroacétate de sodium dans du diméthyl formamide, on ajoute ensuite du chlorure d'oxalyle à une température inférieure à 10° C, puis on chauffe à 60° C. Après refroidissement à environ 0° C on ajoute lentement de la triéthylamine en conservant une température inférieure à 10° C. Les opérations de lavage et d'extraction de l'acroléine sont particulièrement difficiles. Aussi ce procédé extrêmement long et compliqué a-t-il toujours fait reculer l'industrie quant à une production industrielle de fluoromalonaldéhyde par cette voie.

L'industrie pharmaceutique cherche pourtant depuis longtemps une voie simple et économique de préparation des fluoro-malonates ou de leurs sels.

Aussi Mrs ISHIKAWA et TAKAOKA ont proposé dans Chemistry Letters p 107-110 en 1981 un procédé de préparation desdits malonates à partir de l'hexafluoropropène par alcoolyse. Mrs FUCHIKAMI, YAMANOUCHI et SUZUKI ont décrit dans Chemistry Letters p 1573 - 1576 en 1984 un procédé de préparation desdits malonates à partir de chlorotrifluroroéthylène en passant par le sel de lithium de l'acide trifluoroacrylique.

Les matières premières utilisées et les conditions opératoires employées dans ces deux procédés étant onéreuses, elles éliminent toute possibilité d'emploi au niveau industriel.

La présente invention a permis de préparer les fluoromalonates ou leurs dérivés par un procédé tout à fait nouveau à partir de matières premières beaucoup plus facilement accessibles. Elle a permis en outre de synthétiser une classe d'intermédiaires nouveaux que sont les fluoro-1 halogéno-1 dioxa-3,6 bicyclo [4.1.0] heptane de formule générale (I) suivante :

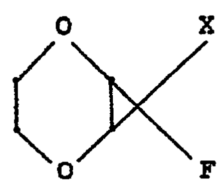

(I)

dans laquelle X représente le chlore, le brome ou le fluor.

On préfère les composés de formule (I) dans lesquels X représente le chlore ou le fluor.

Le procédé de préparation des fluoromalonates selon la présente invention consiste dans une première étape à synthétiser le fluoro-1 halogéno-1 dioxa-3,6 bicyclo [4.1.0] heptane et particulièrement le chloro-1 fluoro-1 dioxa-3,6 bicyclo [4.1.0] heptane. Pour fabriquer ces dérivés on met en présence du dioxène-1,4 ou dioxa-1,4 cyclohexène avec un dihalogénofluoro-méthane de formule $HX_1FCX_2$ dans laquelle $X_1$ et $X_2$ représente un halogène identique (sauf dans le cas du fluor) ou différent choisi parmi le fluor, le chlore ou le brome en milieu basique selon la réaction

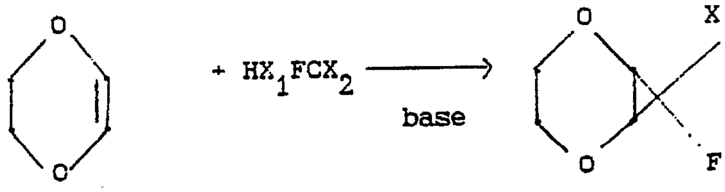


On préfère parmi les dihalogénofluorométhanes utiliser le dichlorofluorométhane ou le chlorodifluorométhane.

Cette réaction est réalisée en présence d'une base minérale forte, éventuellement en solution aqueuse, d'un agent de transfert de phase et éventuellement d'un solvant organique. La base minérale forte peut être choisie parmi les hydroxydes acalins, ou alcalino-terreux. L'agent de transfert de phase peut être choisi parmi les sels d'ammonium, de phosphonium ou parmi les amines tertiaires telles que notamment les trispolyoxaalkylamines et plus particulièrement la tridioxaheptylamine.

Le solvant organique éventuellement utilisé ne doit pas intervenir dans la réaction, on peut citer parmi les solvants utilisables, les halogénométhanes tel que le dichlorométhane et les hydrocarbures aromatiques tels que le benzène, le toluène.....

Les conditions de la réaction du point de vue température ne sont de préférence pas trop sévères. Une température comprise entre 0 et 20° C est préférée. Une pression n'est pas nécessaire pour que la réaction se produise mais les dihalogénofluorométhanes étant gazeux une pression comprise entre 1 et 5 bars est cependant préférée.

A la fin de la réaction le fluoro-1 halogéno-1 dioxa-3,6 bicyclo [4.1.0] heptane est selon les conditions réactionnelles filtré ou extrait par tout solvant organique choisi parmi les éthers, les halogénométhanes ou les hydrocarbures.

Le procédé de préparation selon la présente invention, d'acétals du fluoromalonaldéhyde, composés nouveaux, consiste dans une deuxième étape à mettre en présence le fluoro-1 halogéno-1 dioxa-3,6 bicyclo [4.1.0] heptane préalablement synthétisé avec un acide fort et un alcool ROH selon la réaction suivante

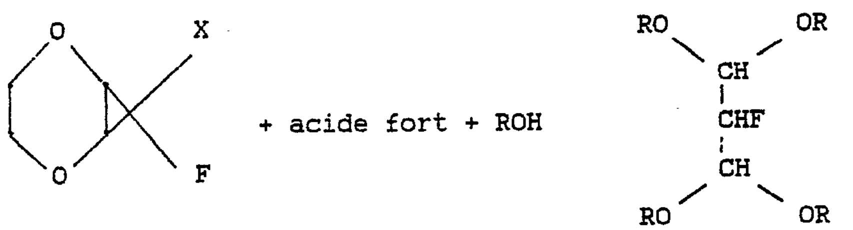


L'alcool de formule ROH est choisi parmi les alcools aliphatiques contenant de préférence 1 à 8 atomes de carbone et encore plus préférentiellement le méthanol et l'éthanol.

L'acide fort est choisi parmi l'acide sulfurique, l'acide trifluoroacétique, l'acide trifluorométhanesulfonique, l'acide chlorhydrique,.....

Les conditions réactionnelles concernant cette deuxième étape seront choisies par l'homme de l'art en fonction de la réactivité de l'acide et de la vitesse de la réaction. On préfère néanmoins opérer à une température comprise entre 40° C et 200° C et de préférence entre 45° C et 120° C pour le méthanol et l'éthanol.

La présente invention vise aussi comme produits nouveaux les bis-dialkylacétals du fluoromalonaldéhyde d'alkyle de formule (II) suivante

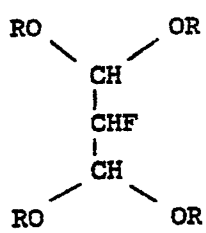

(II)

dans laquelle R à la signification préalablement donnée.

Les bis-dialkylacétals du fluoromalonaldéhyde peuvent être facilement oxydés en fluoromalonates d'alkyle selon toute méthode connue par l'homme de l'art. On peut choisir notamment parmi les agents oxydants un peracide minéral tel que l'acide de Caro constitué d'un mélange de persulfate d'ammonium et d'acide sulfurique à 90 % ou un peracide organique tel que l'acide peracétique.

En ce qui concerne le critère d'utilité des produits nouveaux selon l'invention on peut citer :

L'utilisation des bis-dialkylacétals du fluoromalonaldéhyde directement pour la préparation des dérivés hétérocycliques azotés fluorés sur le cycle tels que les fluoro-4 pyrazoles et les fluoro pyrimidines (obtenus par exemple selon le brevet DE 2 016 990 à partir du fluoromalonaldéhyde).

Les diesters de l'acide fluoromalonique sont notamment utilisés selon l'article de FUCHIKAMI, YAMANOUCHI et SUZUKI paru dans Chemical Letters 1984, p.1573-1576 pour préparer le fluorouracile ou selon l'article de BUCHANAN, DEAN, et PATTISON paru dans Canadian Journal of Chemistry 1962, 40, 1571 pour préparer l'acide $\gamma$ fluoroglutamique.

La présente invention sera plus complètement décrite à l'aide des exemples suivants qui ne doivent pas être considérés comme limitatifs de l'invention.

EXEMPLE 1 - Dioxène-1,4 (préparation)

La préparation de ce composé est effectuée conformément à l'enseignement de l'article de R.P. MOOS, J. PAIGE paru dans J. Chem. Eng. Data, 1967, 12, 452.

EXEMPLE 2 - Préparation du chloro-1 fluoro-1 dioxa-3,6 bicyclo [4.1.0] heptane

Méthode 1 (NaOH aqueux - $CH_2$ $Cl_2$ - TEBA).

Dans un mélange de dioxène-1,4 (12,9 g ; 0,15 mol ; 1 équivalent), de dichlorométhane (15 ml), de soude aqueuse à 50 % en poids (45 ml) et de chlorure de benzyltriéthyl ammonium (TEBA ; 0,05 g), on fait buller du dichlorofluorométhane (23,7 g ; 0,23 mol ; 1,5 équivalent). Le mélange est vigoureusement agité et la température maintenue entre 5 et 10° C. Après l'addition de $CHFCl_2$ le mélange réactionnel est agité pendant 14 heures à 20° C, puis on ajoute de l'eau (300 ml). Les produits sont extraits avec du dichlorométhane (3 x 200 ml). La phase organique est lavée avec une solution saturée de chlorure de sodium puis séchée (sulfate de sodium). Les solvants sont évaporés (20 mm Hg). Le résidu est constitué d'un mélange des deux isomères cis et trans (58 % - 42 %) du cyclopropane fluoré chloré pur (20,8 g ; 0,1365 mol ; rendement 91 %)
Eb :75-76° C / 18 mm Hg.
1H-NMR ($CDCl_3$, TMS) : $\delta$ = 3,6-4,1 ppm (m,4 raies larges).
19 F-NMR ($CDCl_3$, $CFCl_3$) : $\delta$ = -145 ppm (isomère cis, t, 3 JFH = 14 Hz) - 170 ppm (isomère trans, s).
Analyse : calculé pour $C_5$ $H_6$ Cl F $O_2$ (152,55) : C, 39, 36 % ; H,3,96 % ; trouvé : C, 39,76 % ; H, 4, 06 %.

Méthode 2 (NaOH solide - TDA-1)

Dans un mélange, fortement agité, de dioxène-1,4 (6,9 g ; 0,08 mol ; 1 équivalent), de TDA-1 (1,28 g ; 4 $10^{-3}$ mole) et de NaOH en poudre fine (8 g ; 0,2 mol ; 2,5 eq.), on fait buller $CHFCl_2$ (16,5 g ; 0,16 mol ; 2 éq. ) ; la température doit être maintenue entre 5 et 10° C. Le mélange est ensuite agité pendant 14 heures à température ambiante, puis filtré ; le solide gommeux est rincé avec du dichlorométhane (3 x 10 ml). Les phases organiques rassemblées sont évaporées (20 mm Hg). Le résidu est un mélange de dioxène-1,4 et de cyclopropane fluoré chloré qui sont séparés par distillation. On obtient 3,8 g (0,025 mol ; rendement 31 %) de chloro-1 fluoro-1 dioxa-3,6 bicyclo [4.1.0] heptane.

Méthode 3 (NaOH solide - $CH_2$ $Cl_2$ - TDA-1)

On procède comme pour la méthode 2, sur 0,1 mole de dioxène-1,4 en solution dans $CH_2$ $Cl_2$ (50 ml). On obtient après distillation 5,5 g (0,036 mole ; rendement 36 %) de cyclopropane fluoré chloré.

EXEMPLE 3 - A - Obtention du bis-diéthylacétal du fluoromalonaldéhyde

Méthode 4 ($H_2SO_4$ - $C_2H_5OH$ - reflux).

Un mélange de cyclopropane fluoré chloré (6.1 g ; 0.04 mole), d'ethanol absolu (40 ml) et d'acide sulfurique concentré (0,2 ml) est chauffé au reflux pendant 6 jours, puis refroidi et versé sur de l'eau glacée (50 ml). Les produits sont extraits avec de l'éther éthylique (3 x 50 ml). La phase organique est neutralisée avec une solution saturée de bicarbonate de sodium, lavée avec une solution saturée de chlorure de sodium, puis séchée sur du sulfate de sodium. Les solvants sont évaporés (20 mm Hg) et le résidu est distillé pour donner le bis-diéthylacétal du fluoromalonaldéhyde (7,6 g ; 0,032 mol ; rendement 80 %).
Eb : 118-125° C / 12 mm Hg
1H-NMR ($CDCl_3$, TMS) : $\delta$ = 1,25 (t, 12 H, 3JHH = 7Hz) ; 3,7-4,7 ppm (11H).
19 F-NMR ($CDCl_3$, $CFCl_3$) : $\delta$ = -214 ppm (m) ; - 211 ppm (m).
Analyse : calculé pour $C_{11}$ $H_{23}$ $FO_4$ (238,3) : C, 55,44 % ; H, 9,73 % ; trouvé : C, 55,46 % ; H, 9,82 %.

Méthode 5 ($CF_3$ COOH - $C_2H_5OH$ - reflux)

On procède comme dans la méthode 4, l'acide sulfurique concentré étant remplacé par de l'acide trifluoroacétique. On obtient à partir de 1,52 g (0,01 mole) de cyclopropane fluoré chloré, 1,8 g (7,5 $10^{-3}$ mole) de bis-diéthylacétal du fluoromalonaldéhyde.

Méthode 6 ($CF_3SO_3H$ - $C_2H_5OH$ - reflux)

On procède comme dans la méthode 4, l'acide sulfurique concentré étant remplacé par de l'acide trifluorométhane sulfonique. On obtient à partir de 1,52 g (0,01 mole ) de cyclopropane fluoré chloré, 1,9 g (7,9 $10^{-3}$ mole) de bis-diéthylacétal du fluoromalonaldéhyde.

Méthode 7 ($H_2SO_4$ - $C_2H_5OH$ - 120° C)

Un mélange de cyclopropane fluoré chloré (4,6 g ; 0,03 mol), d'éthanol absolu (45 ml) et d'acide sulfurique concentré (0,1 ml ) est placé dans un autoclave de 125 ml, puis chauffé pendant 6 heures à 120° C. Après refroidissement, le traitement du mélange réactionnel est identique à celui décrit dans la méthode 4. On obtient 3,4 g (0,01428 mol ; rendement 47,6 %) de bis-diéthylacétal du fluoromalonaldéhyde.

B - otbention du bis-diméthylacétal du fluoromalonaldéhyde

Méthode 8 ($CF_3COOH$ - $CH_3OH$ - 120° C)

Un mélange de cyclopropane fluoréchloré (3,5 g ; 0,0229 mole), de méthanol (30 ml) et d'acide trifluoroacétique (0,1 ml) est placé dans un autoclave de 125 ml, puis chauffé à 120° C pendant 6 heures. Après refroidissement , le traitement du mélange réactionnel est identique à celui décrit dans la méthode 4. Après évaporation des solvants (20 mm Hg) la distillation du résidu donne le bis-diméthyl-acétal du fluoromalonaldéhyde (1,7 g ; 9,34 $10^{-3}$ mole ; rendement 41 %).
Eb : 86 - 88° C / 17 mm Hg.
1H-NMR ($CDCl_3$, $CFCl_3$) : $\delta$ = 3,5 (s,12 H) ; 4,3-4,8 ppm (m, 3H).
19 F-NMR ($CDCl_3$, $CFCl_3$) : $\delta$ = - 210 ppm (m) ; -213 ppm (m).
Analyse calculé pour $C_7H_{15}FO_4$ (182,194) : C, 46,15 % ; H, 8,30 % ; trouvé : C, 46,25 % ; H,8,17 %.

EXEMPLE 4 - Obtention du fluoromalonate d'éthyle

Méthode 9

A une solution de bis-diéthylacétal du fluoromalonaldéhyde (7,14 g ; 0,03 mole) dans de l'éthanol absolu (60 ml), on ajoute l'acide de Caro (0,15 mole ; 5 équivalents ; préparé à partir de persulfate d'ammonium (34,2 g ; 0,15 mol) et d'acide sulfurique à 90 % (42 g)) . La température est maintenue en dessous de 10° C pendant l'addition. Le mélange est ensuite vigoureusement agité pendant 18 heures à température ambiante ; puis on ajoute de l'eau (200 ml) et on extrait les produits avec de l'éther éthylique (3 x 200 ml). La phase organique est lavée avec une solution saturée de chlorure de sodium puis séchée (sulfate de sodium). Les solvants sont évaporés (20 mmHg). La distillation du résidu donne le fluoromalonate d'éthyle (2,9 g ; 0,0163 mole ; rendement 54 %).
Eb : 90-95° C / 12 mm Hg (littérature : 110-111° C/20 mm Hg (N.Ishikawa, A. Takaoka, Chem.Letters 1981, 107)).

Méthode 10

Un mélange de cyclopropane fluoré chloré (6,1 g ; 0,04 mole), d'éthanol absolu (40 ml), et d'acide sulfurique concentré (0,15 ml) est chauffé au reflux pendant 5 jours, puis refroidi à 0° C. On ajoute l'acide de Caro (0,2 mole ; 5 équivalents ; préparé à partir de persulfate d'ammonium (46,5 g ; 0,2 mole) et d'acide sulfurique à 90 % (56 g)). La température est maintenue en dessous de 10° C pendant l'addition. Le mélange est ensuite vigoureusement agité pendant 18 heures à 20° C puis traité comme dans la méthode 9. On obtient 3,1 g (0,0174 mole ; rendement : 43,5 % ) de fluoromalonate d'éthyle.

EXEMPLE 5 - Condensation du bis-diéthylacétal du fluoromalonaldéhyde avec l'hydrazine : obtention du fluoro-4 pyrazole

Méthode 11

Un mélange de bis-diéthylacétal du fluoromalonaldéhyde (1,2 g ; 0,005 mole), de dichlorure d'hydrazinium (0,525 g ; 0,005 mole), d'eau (0,75 ml) et d'éthanol (0,5 ml) est chauffé au reflux pendant deux heures. Après refroidissement, le mélange est filtré puis on ajoute au filtrat de l'eau (1ml) et du carbonate di-

sodique (1 g). Ce nouveau mélange est filtré. Les produits organiques sont extraits du filtrat avec de l'éther éthylique (2 x 5 ml) ; la phase organique est lavée avec une solution saturée de chlorure de sodium (2 x 5 ml) puis séchée (sulfate de sodium). Les solvants sont évaporés (20 mmHg). Le résidu est du fluoro-4 pyrazole (0,35 g ; 0,004 mole).

**Revendications**

1) Fluoro-1 halogéno-1 dioxa-3,6 bicyclo [4.1.0] heptane de formule (I)

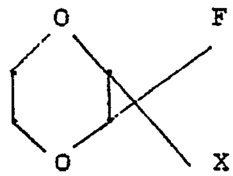

(I)

dans laquelle X représente le fluor, le chlore ou le brome.

2) Chloro-1 fluoro-1 dioxa-3,6 bicyclo [4.1.0] heptane selon la revendication 1

3) Procédé de préparation du composé selon la revendication 1 caractérisé en ce que l'on met au contact en présence d'une base du dioxène-1,4 avec un dihalogénofluorométhane de formule générale $HX_1FCX_2$ dans laquelle $X_1$ et $X_2$ identiques (sauf dans le cas du fluor) ou différents représentent le fluor, le chlore ou le brome.

4). Procédé selon la revendication 3 caractérisé en ce que le dihalogénofluorométhane est le dichlorofluorométhane.

5) Procédé selon la revendication 3 caractérisé en ce que la base est un hydroxyde alcalin ou alcalino-terreux.

6) Procédé selon les revendications 3 et 5 caractérisé en ce qu'on ajoute un agent de transfert de phase.

7) Procédé selon l'une des revendications 3 à 6 caractérisé en ce qu'on ajoute un solvant choisi parmi les halogénométhanes ou les hydrocarbures aromatiques.

8) Procédé selon la revendication 3 caractérisé en ce que la température de réaction est comprise entre 0° C et 20° C.

9) Utilisation des fluoro-1 halogéno-1 dioxa-3,5 bicyclo [4.1.0.] heptanes selon la revendication 1 pour préparer les acétals du fluoromalonaldéhyde caractérisée en ce qu'on met le composé selon la revendication 1 en présence d'un acide fort et d'un alcool de formule ROH.

10) Utilisation selon la revendication 9 caractérisée en ce que l'alcool ROH est choisi parmi les alcools aliphatiques contenant 1 à 8 atomes de carbone.

11) Utilisation selon la revendication 9 caractérisée en ce que l'acide fort est choisi parmi l'acide sulfurique, l'acide chlorhydrique, l'acide trifluoroacétique, l'acide trifluorométhane sulfonique.

12) Utilisation selon la revendication 9 caractérisée en ce que la température de réaction est comprise entre 40° C et 200° C.

13) Acétals du fluoromalonaldéhyde de formule

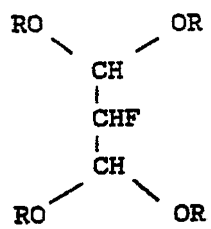

dans laquelle R à la même signification que précédemment

14) Utilisation des acétals du fluoromalonaldéhyde selon la revendication 13 dans la préparation des fluoromalonates caractérisée en ce qu'on oxyde les acétals du fluoromalonaldéhyde par un agent oxydant choisi parmi les péracides minéraux tel que l'acide de Caro, ou parmi les peracides organiques.

15) Utilisation des acétals du fluoromalonaldéhyde selon la revendication 13 dans la préparation d'hétérocycles azotés tels que les fluoro-4 pyrazoles.

**Claims**

1. 1-Fluoro-1-halo-3,6-dioxabicyclo[4.1.0]heptane of formula (I)

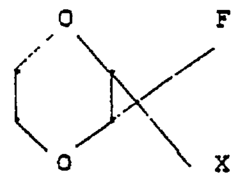

(I)

in which X denotes fluorine, chlorine or bromine.

2. 1-Chloro-1-fluoro-3,6-dioxabicyclo[4.1.0]heptane according to claim 1.

3. Process for preparing the compound according to claim 1, characterized in that 1,4-dioxene is brought into contact, in the presence of a base, with a dihalofluoromethane of general formula $HX_1FCX_2$, in which $X_1$ and $X_2$ may be identical (except in the case of fluorine) or different and denote fluorine, chlorine or bromine.

4. Process according to claim 3, characterized in that the dihalofluoromethane is dichlorofluoromethane.

5. Process according to claim 3, characterized in that the base is an alkali metal hydroxide or alkaline earth metal hydroxide.

6. Process according to claims 3 and 5, characterized in that a phase transfer agent is added.

7. Process according to one of claims 3 to 6, characterized in that a solvent, chosen from halomethanes and aromatic hydrocarbons, is added.

8. Process according to claim 3, characterized in that the reaction temperature is between 0°C and 20°C.

9. Use of 1-fluoro-1-halo-3,6-dioxabicyclo[4.1.0]heptanes according to claim 1 for preparing fluoromalonaldehyde acetals, characterized in that the compound according to claim 1 is brought into contact with a strong acid and an alcohol of formula ROH.

10. Use according to claim 9, characterized in that the alcohol ROH is chosen from aliphatic alcohols containing 1 to 8 carbon atoms.

11. Use according to claim 9, characterized in that the strong acid is chosen from sulphuric acid, hydrochloric acid, trifluoroacetic acid and trifluoromethanesulphonic acid.

12. Use according to claim 9, characterized in that the reaction temperature is between 40°C and 200°C.

13. Fluoromalonaldehyde acetals of formula

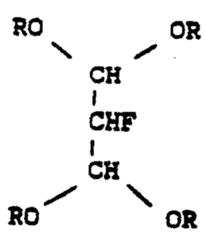

in which R has the same meaning as above.

14. Use of the fluoromalonaldehyde acetals according to claim 13 in the preparation of fluoromalonates, characterized in that the fluoromalonaldehyde acetals are oxidized with an oxidizing agent chosen from inorganic peracids, such as Caro's acid, or from organic peracids.

15. Use of the fluoromalonaldehyde acetals according to claim 13 in the preparation of nitrogencontaining heterocycles such as 4-fluoropyrazoles.

**Patentansprüche**

1. 1-Fluor-1-halogen-3,6-dioxa-bicyclo[4,1,0]heptan der Formel (I)

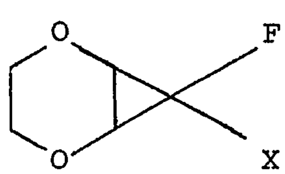

(I)

in der X Fluor, Chlor oder Brom ist.

2. 1-Chlor-1-Fluor-3,6-dioxa-bicyclo[4,1,0]heptan nach Anspruch 1.

3. Verfahren zur Herstellung einer Verbindung nach Anspruch 1, dadurch gekennzeichnet, daß man in Gegenwart einer Base 1,4-Dioxen mit einem Dihalogenfluormethan der allgemeinen Formel

$HX_1FCX_2$

reagieren läßt, in der $X_1$ und $X_2$ identisch (außer bei Fluor) oder verschieden sind und Fluor, Chlor oder Brom bedeuten.

4. Verfahren nach Anspruch 3, dadurch gekennzeichnet, daß das Dihalogenfluormethan Dichlorfluormethan ist.

5. Verfahren nach Anspruch 3, dadurch gekennzeichnet, daß die Base ein Alkali- oder Erdalkalihydroxid ist.

6. Verfahren nach den Ansprüchen 3 und 5, dadurch gekennzeichnet, daß man ein Phasentransfermittel hinzufügt.

7. Verfahren nach einem der Ansprüche 3 bis 6, dadurch gekennzeichnet, daß man ein Lösungsmittel aus der Gruppe der halogenierten Methane oder der aromatischen Kohlenwasserstoffe hinzufügt.

8. Verfahren nach Anspruch 3, dadurch gekennzeichnet, daß die Reaktionstemperatur zwischen 0°C und 20°C liegt.

9. Verwendung von 1-Fluor-1-halogen-3,6-dioxabicyclo[4,1,0]heptanen nach Anspruch 1 zur Herstellung der Acetale des Fluormalonaldehyds, dadurch gekennzeichnet, daß man die Verbindung nach Anspruch 1 mit einer starken Säure und einem Alkohol der Formel ROH versetzt.

10. Verwendung nach Anspruch 9, dadurch gekennzeichnet, daß der Alkohol ROH ein aliphatischer Alkohol mit 1 bis 8 Kohlenstoffatomen ist.

11. Verwendung nach Anspruch 9, dadurch gekennzeichnet, daß die starke Säure Schwefelsäure, Salzsäure, Trifluoressigsäure oder Trifluormethansulfonsäure ist.

12. Verwendung nach Anspruch 9, dadurch gekennzeichnet, daß die Reaktionstemperatur zwischen 40°C und 200°C liegt.

13. Acetale des Fluormalonaldehyds der Formel

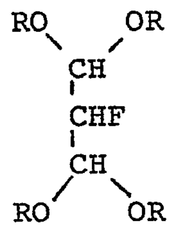

in der R dieselbe Bedeutung wie vorher beschrieben hat.

14. Verwendung von Acetalen des Fluormalonaldehyds nach Anspruch 13 bei der Herstellung von Fluormalonaten, dadurch gekennzeichnet, daß man die Acetale des Fluormalonaldehyds mit einem Oxidationsmittel aus der Gruppe der mineralischen Persäuren, wie z.B. der Carosäure-, oder einer organischen Persäure oxydiert.

15. Verwendung von Acetalen des Fluormalonaldehyds nach Anspruch 13 zur Herstellung von azotierten Heterocyclen wie z.B. 4-Fluorpyrazolen.